(19) Europäisches Patentamt
European Patent Office
Office européen des brevets

(11) Veröffentlichungsnummer: **0 398 217 B1**

(12) **EUROPÄISCHE PATENTSCHRIFT**

(45) Veröffentlichungstag der Patentschrift: **12.01.94**

(51) Int. Cl.$^5$: **A61K 31/59**, C07C 401/00

(21) Anmeldenummer: **90109032.4**

(22) Anmeldetag: **14.05.90**

# (54) Dehydrocholecalciferolderivative.

(30) Priorität: **18.05.89 US 353716**

(43) Veröffentlichungstag der Anmeldung:
**22.11.90 Bulletin 90/47**

(45) Bekanntmachung des Hinweises auf die Patenterteilung:
**12.01.94 Bulletin 94/02**

(84) Benannte Vertragsstaaten:
**AT BE CH DE DK FR GB IT LI LU NL SE**

(56) Entgegenhaltungen:
**EP-A- 0 129 003**
**EP-A- 0 215 956**
**EP-A- 0 307 786**
**EP-A- 0 325 279**

**H.JANISTYN "Handbuch der Kosmetika und Riechstoffe", 3.Auflage, Band 1, 1978 Dr.Alfred HUthig Verlag, Heidelberg Seiten 984-985**

**Klinische Wochenschrift, Band 59, 1981 Springer Verlag K.SCHAEFER, D.VON HERRATH "Vitamin D 1980 - Eine Be- standsaufnahme" Seiten 525-534**

**Abstracts of the ESDR-JSID-SID Tricontinental meeting, Washington, D.C. USA April 26-30 1989, Malloy V.L. et al.**

(73) Patentinhaber: **F. HOFFMANN-LA ROCHE AG**
**Postfach 3255**
**CH-4002 Basel(CH)**

(72) Erfinder: **Baggiolini, Enrico Guiseppe**
**30 Evergreen Drive**
**North Caldwell, N.J. 07006(US)**
Erfinder: **Hennessy, Bernard Michael**
**111 Passaic Avenue**
**Nutley, N.J. 07110(US)**
Erfinder: **Shiuey, Shian-Jan**
**331 Bloomfield Avenue**
**Nutley, N.J. 07110(US)**
Erfinder: **Truitt, Gary Arthur**
**109 Garner Avenue**
**Bloomfield, N.J. 07003(US)**
Erfinder: **Uskokovic, Milan Radoje**
**253 Highland Avenue**
**Upper Montclair, N.J. 07043(US)**

(74) Vertreter: **Mahé, Jean et al**
**Postfach 3255**
**Grenzacherstrasse 124**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft die Verwendung der Dehydrocholecalciferolderivaten der Formel

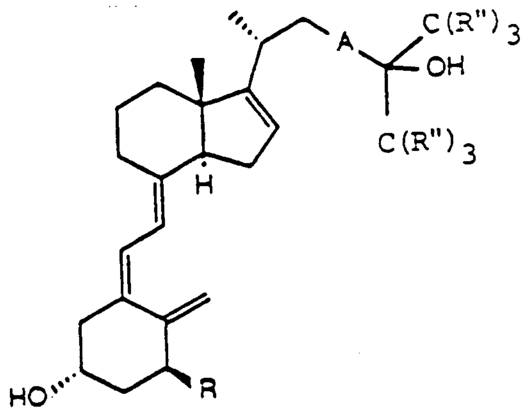

I

worin R H oder OH, A die Gruppierung -C≡C-, -CH = CH-mit E-Konfiguration oder $-CH_2CH_2-$ und R″ H oder, falls A -C≡C- ist, R″ auch Deuterium ist,
bei der Herstellung von pharmazeutischen Präparaten für die Behandlung von mit einer Proliferation der Sebocyten einhergehenden Krankheiten der Talgdrüsen, wie Akne und seborrhoische Dermatitis.

Unter den Verbindungen der Formel I sind diejenigen, worin R″ Deuterium ist, neu und als solche Gegenstand der Erfindung. Die deuterierten Verbindungen können in Analogie zu den nicht-deuterierten Verbindungen, z.B. wie beschrieben in der europäischen Patentanmeldung Nr. 325 279 via Zwischenprodukte der Formel

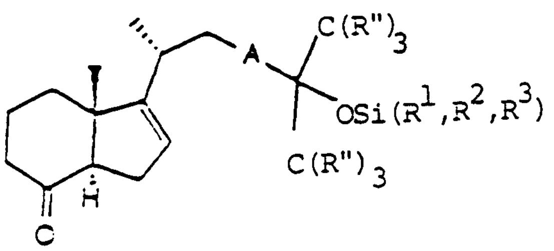

II

worin R″ die obige Bedeutung hat, $R^1$ und $R^3$ nieder-Alkyl und $R^2$ nieder-Alkyl, Aryl oder Aryl-nieder-alkyl sind,
erhalten werden.

Beispiele von Verbindungen der Formel I sind die folgenden Verbindungen A bis H:

A: 1α,25-Dihydroxy-16-dehydrocholecalciferol;
B: 25-Hydroxy-16-dehydrocholecalciferol;
C: 1α,25-Dihydroxy-16,23E-bisdehydrocholecalciferol;
D: 25-Hydroxy-16,23E-bisdehydrocholecalciferol;
E: 1α,25-Dihydroxy-16-dehydro-23-didehydrocholecalciferol;
F: 25-Hydroxy-16-dehydro-23-didehydrocholecalciferol;
G: 26,26,26,27,27,27-Hexadeutero-1α,25-dihydroxy-16-dehydro-23-didehydrocholecalciferol und
H: 26,26,26,27,27,27-Hexadeutero-25-hydroxy-16-dehydro-23-didehydrocholecalciferol.

Die Wirksamkeit von Verbindungen der Formel I bei der Behandlung von Akne kann man wie folgt zeigen:

Sebocyten wurden aus Talgdrüsen des Erwachsenen in Analogie zu der in Cells 6, 1975, 331-334 und In Vitro 22:3, 1986, II, p. 22a, Abstract 46 beschriebenen Methode isoliert und auf einer Schicht von Mäusen-Fibroblasten 3T3 kultiviert. Durch enzymatische und mechanische Methoden wurde aus Hautgewebe eine Suspension von Sebocyten hergestellt. Dazu wurden die Zellen entweder in 2% Humanserum, 8% fetales Kälberserum und 4 µg/ml Dexamethason oder in 10% fetales Kälberserum und 4 µg/ml Dexamethason enthaltendem Iscove-Medium kultiviert

Den Kulturen wurden die Testverbindungen im Medium alle 48 Stunden zugegeben. Nach der letzten Zugabe wurden die Fibroblasten 3T3 mit 0,03% EDTA in PBS abgespült. Die zurückbleibenden Sebocyten-Kolonien wurden in 0,05% Trypsin/0,03% EDTA unter Bildung einer einheitlichen Zellensuspension von Sebocyten inkubiert. Die Zellen wurden dann verdünnt und mittels eines Hämocytometers gezählt.

Es wurden $10^{-2}$ M äthanolische Lösungen der Testverbindungen vorbereitet und dann auf $10^{-6}$, $10^{-7}$, $10^{-8}$ und $10^{-9}$ M verdünnt. Zusammen mit den Verbindungen der Formel I wurde auch das 1α,25-Dihydroxycholecalciferol (Verbindung X in den nachstehenden Tabellen) auf eine Hemmung der Proliferation von Sebocyten in vitro getestet.

Die Resultate sind in der nachstehenden Tabelle 1 als Menge der Verbindung, die die Proliferation der Sebocyten um 50% gegenüber einem Kontrollwert hemmen, angegeben. Der Kontrollwert wurde aus einer lediglich mit Lösungsmittel behandelten Zellenkultur erhalten.

Tabelle 1

| Verbindung | X | A | D | E | F |
|---|---|---|---|---|---|
| $ED_{50}$ (μM) | 0,005 | 0,001 | >1 | 0,001 | 0,1 |

Die Resultate zeigen, dass die Verbindungen der Formel I die Proliferation der menschlichen Sebocyten in vitro hemmen und daher als Mittel für die Behandlung von Akne verwendbar sind.

Die topische Wirksamkeit von Verbindungen der Formel I gegen Akne wurde an Talgdrüsen des Hamsterohrs gemessen. Versuchstieren wurden 50 μl einer Lösung der Testverbindung und Kontrolltieren 50 μl Aceton täglich auf das Ohr appliziert. Nach 4 Wochen wurden die Tiere getötet. Die Fläche der Talgdrüsen auf den Ohren wurde ermittelt. In der Tabelle 2 sind die Resultate als %-Aenderung gegenüber Kontrolltieren der Veränderung des Querschnitts der Talgdrüsen auf dem Hamsterohr angegeben:

Tabelle 2

| Verbindung | Dosis (μg/Hamster) | % Aenderung |
|---|---|---|
| D | 0,10 | -16 |
| | 1,00 | -16 |
| | 10,00 | -43 |
| F | 0,01 | - 8 |
| | 0,10 | -23 |
| | 1,00 | -40 |
| | 10,00 | -64 |

Zum Nachweis der Verkalkung von weichem Gewebe durch die Verbindungen der Formel I erhielten Ratten subkutane Injektionen von 40 μCi $^{45}Ca$. Die Verbindungen wurden dann subkutan oder topisch während 4 Tagen den Tieren verabreicht. Die Ratten wurden 1 Tag nach der letzten Injektion getötet. Herz und Nieren wurden entfernt und mit Salpetersäure behandelt. Es wurde die Radioaktivität einer Menge von 0,2 ml dieser Präparation gemessen. In der Tabelle 3 ist das Verkalkungsverhältnis

$$\frac{\text{(cpm von X - cpm der Kontrolle)}}{\text{(cpm der Testverbindung - cpm der Kontrolle)}}$$

angegeben:

Tabelle 3

| Verbindung | Subkutan | Topisch |
|---|---|---|
| X | 1 | 1 |
| D | >1400 | >34 |
| E | 47 | > 1 |
| F | >1400 | >34 |

Der Effekt von Verbindungen der Formel I auf die Talgdrüsen des Hamsterohrs nach oraler Verabreichung wurde ermittelt. Männlichen syrischen Goldhamstern wurden täglich 200 µl einer Lösung der Testverbindungen in Propylenglykol oral verabreicht. Nach 4 Wochen wurden die Tiere getötet. Die Fläche der Talgdrüsen auf den Ohren wurde ermittelt. Die Resultate sind in Tabelle 4 als Veränderung im Querschnitt der Talgdrüsen des Hamsterohrs angegeben:

Tabelle 4

| Verbindung | Dosis (µg/kg) | % Veränderung |
|---|---|---|
| D | 2,50 | -15* |
| | 5,00 | -22** |
| | 10,00 | -27*** |
| | 20,00 | -36*** |
| F | 0,05 | -16* |
| | 0,50 | -23** |
| | 5,00 | -42*** |
| | 50,00 | -55*** |

* p <0,05;
** p <0,01;
*** p <0,001

Die obigen Resultate zeigen, dass Verbindungen der Formel I verwendbar sind als Wirkstoffe in der Behandlung von Krankheiten der Talgdrüsen, wie Akne oder seborrhoische Dermatitis und ferner dass sie weniger Verkalkung der weichen Gewebe als 1α,25-Dihydroxycholecalciferol verursachen. Die Verkalkung der weichen Gewebe ist eine unerwünschte Nebenwirkung eines Mittels zur Behandlung von Krankheiten der Talgdrüsen.

Die Verbindungen der Formel I können topisch oder oral zur Behandlung von Krankheiten der Talgdrüsen, wie Akne oder seborrhoische Dermatitis, verabreicht werden. So können sie in einer oralen Dosis von etwa 0,7 bis 700, vorzugsweise 7,0 bis 70 µg pro Tag zur oralen Behandlung von Akne verabreicht werden.

Zur oralen Verabreichung können die Verbindungen der Formel I z.B. in Kapseln oder Tabletten zusammen mit pharmazeutisch verwendbaren Trägern verarbeitet werden. Beispiele von solchen Trägern für Kapseln sind Bindemittel, wie Tragacanth, Gummi oder Gelatine; Excipientien, wie Dicalciumphosphat; Sprengmittel, wie Maisstärke; Gleitmittel, wie Magnesiumstearat; Süsstoffe, wie Sucrose; Aromastoffe, wie Pfefferminz. Tabletten können mit Shellack, Zucker oder beidem überzogen sein. Ein Sirup oder Elixir kann einen Süssstoff, Methyl- und Propylparabene als Konservierungsmittel, ein Farbstoff und einen Aromastoff enthalten.

Topische Dosierungsformen enthaltend Verbindungen der Formel I sind Salben und Crème bestehend aus öligen, resorbierbaren, wasserlöslichen und emulsionsartigen Basen, wie Lanolin und Poyläthylenglykol. Weitere topische Dosierungsformen sind Gele, Lotionen, Pulver und Aerosole.

Lotionen d.h. flüssige Präparate wie Lösungen bzw. wässrige oder hydroalkoholische Präparate, die die Substanz in Pulverform enthalten, können Suspendierungs- oder Dispergiermittel, wie Cellulosederivate, z.B. Aethyl- oder Methylcellulose; Gelatine oder Gummi, sowie ein Vehikel, wie Wasser, Alkohol oder Glycerol, neben dem Wirkstoff enthalten. Gele sind halbfeste Präparate, die aus einer Lösung oder einer Suspension des Wirkstoffs in einem Vehikel hergestellt werden. Die wässrigen oder wasserfreien Vehikel werden mit einem Gelierungsmittel behandelt, z.B. Carboxypolymethylen, und mit einer Base neutralisiert,

z.B. Natriumhydroxid oder einem Amin wie Polyäthylencocoamin. In den folgenden Beispielen ist die Zusammensetzung von Weichgelatinekapseln für die orale Verabreichung und von einer topisch verabreichbaren Crème angegeben:

| Beispiel A | |
|---|---|
| | mg/Kapsel |
| Verbindung E | 0,0001-0,010 |
| Butyliertes Hydroxytoluol | 0,016 |
| Butyliertes Hydroxyanisol | 0,016 |
| Fraktioniertes Kokosnussöl | 160,0 |

| Beispiel B | |
|---|---|
| | mg/g Crème |
| Verbindung E | 0,001-1,0 |
| Cetylalkohol | 1,5 |
| Stearylalkohol | 2,5 |
| Sorbitanmonostearat | 2,0 |
| Glycerylmonostearat und Polyoxyäthylenglycolstearat | 4,0 |
| Polysorbat 60 | 1,0 |
| Mineralöl | 4,0 |
| Propylenglykol | 5,0 |
| Propylparaben | 0,05 |
| Butyliertes Hydroxyanisol | 0,05 |
| Sorbitollösung | 2,0 |
| EDTA-Dinatriumsalz | 0,01 |
| Methylparaben | 0,18 |
| Destilliertes Wasser | q.s. zu 100 g |

## Patentansprüche

**1.** Verwendung der Dehydrocholecalciferolderivaten der Formel

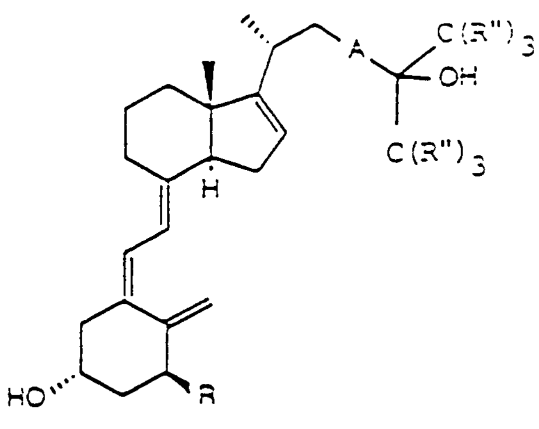

I

worin R H oder OH, A die Gruppierung -C≡C-, -CH = CH-mit E-Konfiguration oder $-CH_2CH_2-$ und R'' H oder, falls A -C≡C- ist, R'' auch Deuterium ist,
bei der Herstellung von pharmazeutischen Präparaten für die Behandlung von mit einer Profileration der Sebocyten einhergehenden Krankheiten der Talgdrüsen.

**2.** Verwendung nach Anspruch 1, worin die zu behandelnde Krankheit Akne ist.

**3.** Verbindungen der Formel I in Anspruch 1, worin R" Deuterium ist.

## Claims

**1.** The use of dehydrocholecalciferol derivative of the formula

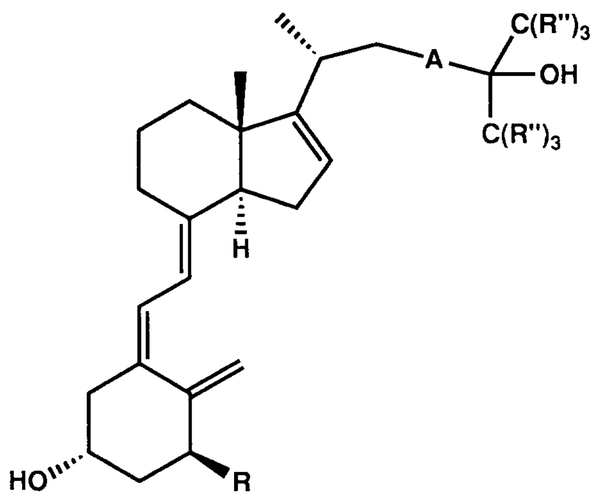

I

wherein R is H or OH, A is -C≡C-, -CH = CH- with the E-configuration or $-CH_2CH_2-$ and R" is H or, where A is -C≡C-, R" is also deuterium, for the manufacture of pharmaceutical preparations for the treatment of sebaceous gland diseases which are associated with a proliferation of sebocytes.

**2.** The use according to claim 1, wherein the disease to be treated is acne.

**3.** Compounds of formula I in claim 1, wherein R" is deuterium.

## Revendications

**1.** Utilisation de dérivés du déshydrocholécalciférol de formule

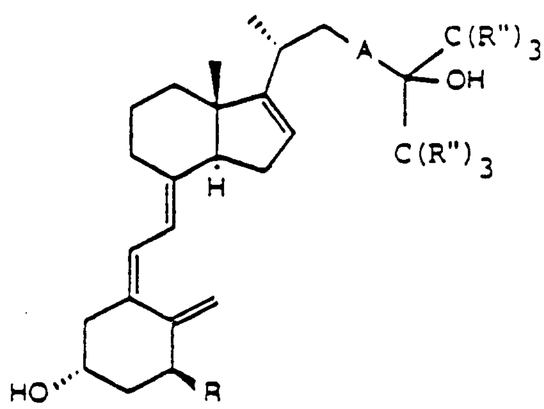

I

dans laquelle R représente H ou OH, A représente le groupement -C≡C-, -CH = CH- en configuration E ou $-CH_2CH_2-$ et R" représente H ou bien encore, lorsque A représente -C≡C; le deutérium, pour la préparation de compositions pharmaceutiques servant au traitement des maladies des glandes sébacées s'accompagnant d'une prolifération des sébocytes.

2. Utilisation selon la revendication 1, dans laquelle la maladie à traiter est l'acné.

3. Composés de formule I de la revendication 1, dans laquelle R'' représente le deutérium.